Europäisches Patentamt

European Patent Office

Office européen des brevets

(11) Publication number: **0 213 632 B1**

## (12) EUROPEAN PATENT SPECIFICATION

(45) Date of publication of patent specification: 17.01.90

(51) Int. Cl.⁵: **A 61 K 7/48**

(21) Application number: 86112136.6

(22) Date of filing: 02.09.86

(54) Moisture-retaining preparation for dermal use.

(30) Priority: 05.09.85 JP 196722/85

(43) Date of publication of application:
11.03.87 Bulletin 87/11

(45) Publication of the grant of the patent:
17.01.90 Bulletin 90/03

(84) Designated Contracting States:
DE FR GB IT

(56) References cited:
PATENT ABSTRACTS OF JAPAN, vol. 9, no. 17
(C-262)1740r, 24th January 1985; & JP-A-59 164
711 (HIDEAKI TOUYAMA) 17-09-1984

PATENT ABSTRACTS OF JAPAN, vol. 5, no. 156
(C-74)828r, 6th October 1981; & JP-A-56 86 111
(HIROYO ENDOU) 13-07-1981

PATENT ABSTRACTS OF JAPAN, vol. 9, no. 1
(C-259)1724r, 5th January 1985; & JP-A-59 154
971 (ZENZOU NAKAGIRI) 04-09-1984

(73) Proprietor: Yoshida, Shoji
32-318, Kurakuen 5-Bancho 2-chome
Nishinomiya-shi Hyogo 662 (JP)
(73) Proprietor: Ogata, Kazumi
B-701, 8, Kamishinden 4-chome
Toyonaka-shi Osaka 565 (JP)

(72) Inventor: Ogata, Kazumi
B-701, 8. Kamishinden 4-chome
Toyonaka-shi Osaka 565 (JP)
Inventor: Tsuruoka, Hideki
202-39, Aza-Nor Higashitada
Kawanishi-shi Hyogo 666-01 (JP)

(74) Representative: von Kreisler, Alek, Dipl.-Chem.
et al
Deichmannhaus am Hauptbahnhof
D-5000 Köln 1 (DE)

Courier Press, Leamington Spa, England.

**Description**

BACKGROUND OF THE INVENTION

1. Field of the Invention

This invention relates to a preparation to be applied to the skin which contains sake essence as a moisture-retaining agent.

2. Description of the Prior Art

Moisture-retaining agents are essential components in preparations to be applied to the skin, such as ointments, creams, gels and lotions, in order to maintain the moisture content of the skin at a constant level.

Glycerin, sorbitol, aloe extract and placenta extract are known examples of moisture-retaining agents.

Glycerin and sorbitol are inexpensive and good synthetic moisture-retaining agents. On the contrary, the aloe extract, an extract from a natural product, has a drawback that it causes discoloration of preparations and the placenta extract, another nature-origin moisture-retaining agent, is difficult to obtain and very expensive. Thus, no satisfactory moisture-retaining agents of natural origin have yet been known.

Under these circumstances, inexpensive and safe moisture-retaining agents of natural origin which are free of such drawbacks as mentioned above have been searched for in recent years.

Accordingly, the present inventors made intensive investigations in search of a moisture-retaining agent which is of natural origin and excellent in quality and, as a result, found that the residue after distillation of sake, namely the so-called sake essence, has potent moisture-retaining property. The present invention has been completed on the basis of this finding.

From Pat. abstr. Jap., Vol. 9, No. 17 (C-262) (1740), 1985 a cosmotic composition for preventing skin roughness is known which contains refined sake, sake lees, rice bran and wheat flour etc. Furthermore, Pat. abstr. Jap., Vol. 5, No. 156 (C-74) (828), 1981 describes a lemon liquid for cosmetic use containing refined sake.

SUMMARY OF THE INVENTION

However, the present invention consists in incorporating sake essence, obtained after removal from sake of volatile components as a moisture-retaining agent into preparations to be applied to the skin, such as creams, gels, ointments and lotions.

DETAILED DESCRIPTION OF THE INVENTION

In accordance with the invention, sake essence is used for the same purposes as in the case of moisture-retaining agents generally incorporated into preparations for dermal use. Therefore, there are no particular limitations or restrictions as to the other components of such preparations than the moisture-retaining agent. Furthermore, sake essence can be used in combination with other moisture-retaining agents.

The term "preparation for dermal use" as used herein includes, within the meaning thereof, pharmaceutical or cosmetic preparations to be applied to the skin, whether for medical purposes or not, such as, for example, ointments, gels, packs, lotions and skin creams.

The term "sake essence" as used herein means a syrupy residual concentrate or a powder obtained after removal from sake of volatile components, such as alcohols, by distillation and a lyophilizate obtained from such concentrate.

The term "sake" as used herein means a wine made from rice or starch as the main raw material by alcoholic fermentation.

In the experiment, sake essence and other moisture-retaining agents were compared with respect to their moisture-absorbing and moisture-retaining properties. Thus, 1 g of each sample was placed in a 5-ml beaker and allowed to stand in a constant-temperature, constant-humidity chamber (30°C, 90% or 38% RH), with sample weighing at timed intervals. The sake essence used in this experiment was a powder obtained by lyophilization of a sake essence concentrate, which in turn was obtained from second-grade sake (produced by Tamon Shuzo Kabushiki Kaisha) by concentration under reduced pressure, and this powder was dissolved in water to give a 70% (W/V) solution. Analytical data indicated that the carbohydrate content of said powder essence was approximately 90% and the protein content approximately 7.4%.

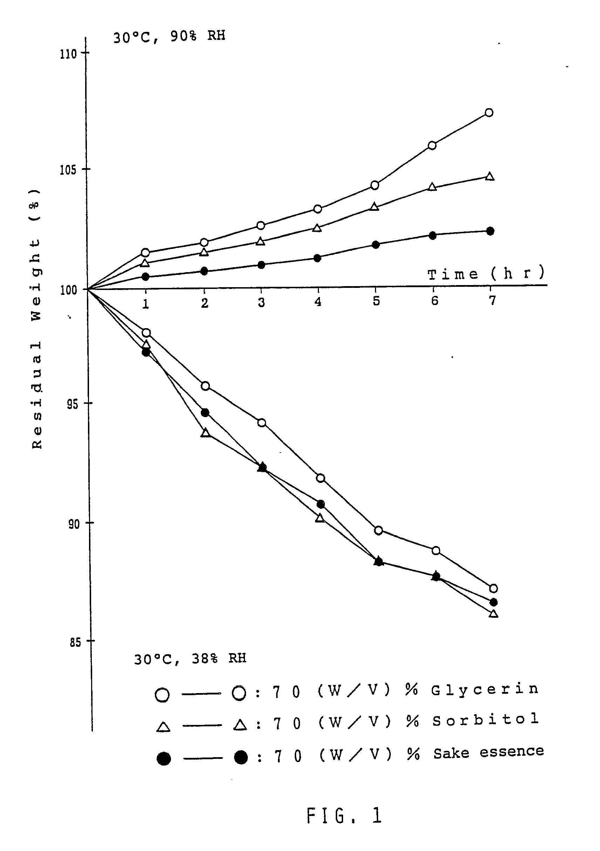

The results obtained in said experiment are graphically shown in Fig. 1 (moisture-retaining effect curve).

As is evident from Fig. 1, the increase in water content, namely the hygroscopicity, was less with the 70% (W/V) sake essence than with 70% (W/V) glycerin or sorbitol and the loss in water, namely the moisture-retaining effect, was substantially the same between the 70% sake essence and 70% glycerin or sorbitol.

The above results indicate that sake essence is sufficiently effective as a moisture-retaining agent for use in preparations for dermal use.

Moreover, sake essence to be used as a moisture-retaining agent in accordance with the invention is advantageous over glycerin or sorbitol in that it contains a number of skin-nourishing ingredients such as carbohydrates, proteins, amino acids and vitamins.

In a blindfold test using a panel of 10 women, three kinds of cosmetic creams, namely the cream of Example 5 with sake essence incorporated therein and two corresponding creams with 5% of sorbitol and 5% of glycerin respectively incorporated therein in lieu of 5% of sake essence were comparatively evaluated with respect to impressions after use thereof.

The creams were evaluated in the five aspects given below and scored in the manner mentioned below.

Aspects:
(1) Moisturizing effect,
(2) Nonstickiness,
(3) Extendability,
(4) Affinity for the skin, and
(5) Foundation (for make-up) effect.

Scores:
3 for the first cream in the order of preference,
2 for the second, and
1 for the third.
The result thus obtained are shown in Table 1 in terms of the total score given by the 10 panelists.

TABLE 1

| Aspect | Example 5 (5% sake essence) | 5% sorbitol | 5% glycerin |
|--------|------------------------------|-------------|-------------|
| (1) | 29 | 17 | 14 |
| (2) | 27 | 16 | 17 |
| (3) | 30 | 15 | 15 |
| (4) | 30 | 15 | 15 |
| (5) | 28 | 16 | 16 |

It was thus found that the formulation of Example 5, which contains 5% of sake essence, is superior in every aspect to the formulation using, as the moisture-retaining agent, 5% of sorbitol and 5% of glycerin, respectively.

In the above test, no panelists complained of dermal irritation, pain, nor itching. Further, there could not be found any congestion nor swelling at the application sites of the panelists. Thus, it is evident that all of these moisture-retaining agents including sake essence have no harmful effect to human skin.

The concentration of sake essence in preparations may vary depending on the final form of preparations, the mode of use, and so forth. Generally, however, it is recommended that sake essence be used in a concentration of 0.1 to 40 (W/V) percent, preferably to 1.0 to 30 (W/V) percent, as calculated on the sake essence solids basis.

When intended for medical use, the preparations for dermal use which contain sake essence in accordance with the invention may naturally contain one or more pharmacologically active ingredients. When intended for cosmetic use, they may contain various materials in common use in cosmetic preparations. The preparations may further contain surfactants (e.g. polyoxyethylene-sorbitan monooleate, polyoxyethyleneglycerin monostearate, glycerin monostearate, sorbitan monooleate, propylene glycol monostearate, polyethylene glycol monostearate, polyoxyethylene lauryl ether, polyoxyethylenepolyoxy-propylene cetyl ether, polyoxyethylene nonylphenyl ether, polyoxyethylenehydrogenated castor oil, sodium lauryl sulfate, sodium polyoxyethylene oleyl ether phosphate), preservatives (e.g. benzalkonium chloride, benzethonium chloride, cetylpyridinium chloride, chlorobutanol, p-hydroxybenzoic acid esters, cetyltrimethylammonium bromide, sorbic acid) and pH-adjusting agents (e.g. citric acid, lactic acid, carbonic acid, phosphoric acid, acetic acid, salts of these, sodium hydroxide, potassium hydroxide, hydrochloric acid), without any impairment thereof. Furthermore, they may contain appropriate fragrances and colorants, as desired.

The following examples are further illustrative of the present invention. The sake essence (powder) used therein was prepared by concentrating 1 liter of second-grade sake (produced by Tamon Shuzo) under reduced pressure and lyophilizing the essence concentrate thus obtained. Yield 60 g (6%). The "sake essence, 70 (W/V) percent" as so referred to in the example, was prepared by adding water to the powder-form essence to an essence concentration of 70 (W/V) percent.

Example 1

Ointment

| | |
|---|---|
| Zinc white | 25 g |
| Talc | 10 g |
| Vitamin A palmitate | $1 \times 10^5$ IU |
| Vitamin $D_3$ | $4 \times 10^4$ IU |
| Sake essence (powder) | 20 g |
| Carboxymethylcellulose sodium | 1.0 g |
| Olive oil | 5.0 g |
| Glycerin monostearate | 2.0 g |
| Methyl p-hydroxybenzoate | 0.025 g |
| Butyl p-hydroxybenzoate | 0.015 g |
| Purified water | To make 100 g |

The above ingredients are blended by the conventional method to give an ointment.

Example 2

Gel

| | |
|---|---|
| *l*-Menthol | 1.5 g |
| *dl*-Camphor | 1.0 g |
| Dipotassium glyzyrrhizinate | 0.1 g |
| LAUROMACROGOL® | 2.0 g |
| Sake essence, 70 (W/V) percent | 5.0 g |
| Carboxyvinyl polymer | 1.0 g |
| Triethanolamine | 1.5 g |
| Ethanol | 35 ml |
| Purified water | To make 100 g |

The above ingredients are blended in the conventional manner to give a gel.

Example 3

Pack

| | |
|---|---|
| Polyvinyl alcohol | 15 g |
| Sake essence, 70 (W/V) percent | 10 g |
| Ethanol | 10 ml |
| Methyl p-hydroxybenzoate | 0.028 g |
| Butyl p-hydroxybenzoate | 0.012 g |
| Purified water | To make 100 g |

The above ingredients are blended in the conventional manner to give a pack.

# EP 0 213 632 B1

## Example 4

### Cosmetic lotion

| | |
|---|---|
| Sake essence, 70 (W/V) percent | 5.0 g |
| Citric acid | 0.5 g |
| Polyoxyethylene oleyl ether | 1.0 g |
| Ethanol | 10.0 ml |
| Methyl p-hydroxybenzoate | 0.028 g |
| Butyl p-hydroxybenzoate | 0.012 g |
| Purified water | To make 100 g |

The above ingredients are blended in the conventional manner to give a cosmetic lotion.

## Example 5

### Cosmetic cream

| | |
|---|---|
| Squalane | 10.0 g |
| Isopropyl myristate | 5.0 g |
| White beeswax | 3.0 g |
| Stearyl alcohol | 3.0 g |
| Polyethylene glycol monostearate | 2.0 g |
| Glycerin monostearate | 4.0 g |
| Vitamin E acetate | 0.2 g |
| Sake essence (powder) | 5.0 g |
| Methyl p-hydroxybenzoate | 0.2 g |
| Butyl p-hydroxybenzoate | 0.1 g |
| Purified water | To make 100 g |

The above ingredients are blended in the conventional manner to give a cosmetic cream.

## Claims

1. A preparation for dermal use which contains sake essence obtained after removal from sake of volatile components as a moisture-retaining agent.

2. A preparation for dermal use as claimed in Claim 1, wherein the concentration of sake essence is 0.1—40 (W/V) percent on the sake essence solids basis.

## Patentansprüche

1. Präparat zur dermalen Anwendung, enthaltend Sake-Essenz, die durch Entfernen flüchtiger Bestandteile aus Sake erhalten worden ist, als feuchtigkeitszurückhaltendes Mittel.

2. Präparat zur dermalen Anwendung nach Anspruch 1, worin die Konzentration der Sake-Essenz 0,1 bis 40 Gew./Vol.-%, bezogen auf den Feststoff-Gehalt der Sake-Essenz, beträgt.

## Revendications

1. Préparation pour utilisation dermique, qui contient, à titre d'agent de retenue de l'humidité, de

5

l'essence de saké obtenue après enlèvement des composants volatils du saké.

2. Préparation pour utilisation dermique, telle que revendiquée à la revendication 1, dans laquelle la concentration de l'essence de saké est de 0,1 à 40% en poids/volume, sur la base des solides de l'essence de saké.

FIG. 1